# EUROPÄISCHE PATENTANMELDUNG

(11) **EP 4 520 250 A1**
(43) Veröffentlichungstag der Anmeldung: **12.03.2025**
(21) Anmeldenummer: 24197882.4
(22) Anmeldetag: 02.09.2024
(51) Int. Cl.: A61B 1/00, A61B 1/05, G02B 23/24

(54) **ENDOSKOP MIT EINEM STARREN ODER FLEXIBLEN SCHAFTROHR**

(30) Priorität: 06.09.2023 DE 102023123953
(71) Anmelder: Karl Storz SE & Co. KG, 78532 Tuttlingen (DE)
(72) Erfinder: Reinacher, Joachim, 78532 Tuttlingen (DE)

(57) **Zusammenfassung**

Die Erfindung betrifft ein Endoskop mit einem starren oder flexiblen Schaftrohr, mit einer Rotationstrommel (1) welches an einem distalen Ende, bevorzugt an einer Lagergabel (9), um wenigstens eine Rotationsachse rotierbar gelagert ist, wobei die Rotationstrommel (1) eine Ausnehmung umfasst, in welcher ein zugeordnetes Abbildungssystem wenigstens teilweise aufgenommen wird, welches zumindest eine Abbildungsoptik (5) und einen Bildsensor (6) umfasst, wobei das Abbildungssystem durch eine, bevorzugt flexible, Leiterplatte (7) mit dem proximalen Bereich des Endoskops leitend verbunden ist, und wobei das Endoskop zumindest ein Steuermittel (8) aufweist, um die Rotationstrommel (1) zu bewegen, insbesondere um die wenigstens eine Rotationsachse zu drehen. Erfindungsgemäß ist vorgesehen, dass die Rotationstrommel (1) einstückig, bevorzugt monolithisch, ausgeführt ist und wenigstens einen, bevorzugt federnden, Steuerfortsatz (3) aufweist.

## Beschreibung

Die vorliegende Erfindung betrifft ein Endoskop mit einem starren oder flexiblen Schaftrohr, mit einer Rotationstrommel.

Endoskope mit einem drehbaren Kopfteil sind im Stand der Technik in unterschiedlichen Ausgestaltungen grundsätzlich bekannt. Aus der DE 10 2020 132 773 B3, DE 10 2020 132 776 A1, DE 10 2020 132 778 A1 der Anmelderin sind Endoskope mit einer Rotationstrommel bekannt, wobei die Rotationstrommel jeweils über eine Steuerleitung, welche außen am Schaft in einem Arbeitskanal verläuft, rotiert wird.

Aus der EP 1 759 629 A1 der Anmelderin ist ein Endoskop mit rotierbarer Kameraaufnahme bekannt, wobei über einen elektrischen Motor eine Zahnradvorrichtung angetrieben wird, welche zur Rotation der Kameraaufnahme führt. Alternativ kann die Kameraaufnahme über eine Riemenanordnung rotiert werden. Eine Rotation der Kameraaufnahme über eine Riemenanordnung ist ebenso aus der WO 2017/040692 A1 bekannt.

Aus der EP 3 243 426 A1 der Anmelderin ist ein Endoskop bekannt, bei dem die Kamera entlang einer Schiene bewegt werden kann um das Sichtfeld einzustellen.

Aus der US 2015/0359420 A1 ist ein Endoskop bekannt, wobei die Kameraaufnahme an zwei Achszapfen gelagert ist und Rotationskraft über ein Torsionsrohr an die schwenkbare Kameraaufnahme übertragen wird.

Aus der D10 2012 206 963 A1 ist ein Endoskop mit drehbar gelagerter Kameraaufnahme bekannt, wobei die Rotation über eine antreibbare Rolle bewirkt wird, welche handbetätigt werden kann oder über ein Zugmittelgetriebe gesteuert wird.

Aus der DE 10 2005 015 522 A1 ist eine intrakorporale Sonde mit einem Gehäuse bekannt, in dem sich eine Bildaufnahmeeinheit befindet. Die Bildaufnahmeeinheit ist im Gehäuse schwenkbar, um durch diese Bewegung einen Aufnahmebereich der Bildaufnahmeeinheit zu verändern. Ein mit der Bildaufnahmeeinheit gekoppelter Motor führt die Bewegung aus.

In der US 2022/248946 A1 ist ein Otoskop gezeigt, welches eine Sonde und eine Kamera mit einem Bildsensor in einem Gehäuse umfasst, welche an einem Sondenkörper der Sonde angeordnet ist. Die Kamera ist gegenüber dem Sondenkörper rotierbar gelagert. Das Otoskop umfasst ferner Kabel, die die Rotation der Kamera ermöglichen.

Nachteilig am bisher bekannten Stand der Technik ist, dass die bekannten Lösungen teilweise sehr platzintensiv ausgebildet werden und oft aus vielen einzelnen Bauteilen bestehen, wodurch die Fertigung und Installation anspruchsvoll und fehleranfällig und schwer zu automatisieren ist.

Vor diesem Hintergrund besteht die Aufgabe der vorliegenden Erfindung darin, die Nachteile im Stand der Technik zu überwinden, insbesondere ein Endoskop mit Rotationstrommel vorzuschlagen, welche besonders platzsparend und einfach im distalen Ende des Endoskops installiert werden kann.

Diese Aufgabe wird mit einem Endoskop mit den Merkmalen des Anspruchs 1 gelöst.

Vorteilhafte Ausführungsformen des erfindungsgemäßen Endoskops sind Gegenstand der nachfolgenden Beschreibung und Figurenbeschreibung sowie der Unteransprüche.

Die vorrichtungsmäßig beschriebenen und beanspruchten Merkmale sollen auch als entsprechend verfahrensmäßig offenbart und beanspruchbar gelten, und umgekehrt.

Das Endoskop sieht, wie oben bereits eingeführt, ein starres oder flexibles Schaftrohr vor, mit einer Rotationstrommel, welches an einem distalen Ende, bevorzugt an einer Lagergabel, um wenigstens eine Rotationsachse rotierbar gelagert ist.

Die Definition von distal und proximal bezieht sich auf den Anwender des Endoskops. Distal bedeutet dementsprechend, das am weitesten vom Anwender entfernte Ende des Endoskops, bei einem chirurgischen Eingriff also das Ende was als erstes in die Operationsöffnung eingeführt wird. Das proximale Ende ist das Ende, welches am nächsten dem Anwender zugewendet ist, zum Beispiel das Ende an welchem das Endoskop gehalten oder geführt wird.

Die Rotationstrommel umfasst dabei eine Ausnehmung, in welcher ein zugeordnetes Abbildungssystem wenigstens teilweise aufgenommen wird, welches zumindest eine Abbildungsoptik und einen Bildsensor umfasst wobei das Abbildungssystem durch eine, bevorzugt flexible, Leiterplatte mit dem proximalen Bereich des Endoskops leitend verbunden ist.

Weiterhin weist das Endoskop zumindest ein, bevorzugt mechanisches, Steuermittel auf, um die Rotationstrommel zu bewegen, insbesondere um die wenigstens eine Rotationsachse zu drehen.

Erfindungsgemäß ist dabei vorgesehen, dass die Rotationstrommel einstückig, bevorzugt monolithisch, ausgeführt ist und wenigstens einen, bevorzugt federnden, Steuerfortsatz aufweist.

Die vorliegende Erfindung hat erkannt, dass durch die einstückige Ausführung der Rotationstrommel, verschiedene Probleme gelöst werden können, wodurch insgesamt eine kompakte Ausführung erreicht wird, die wenig Platz im distalen Ende des Endoskops einnimmt und leicht installiert werden kann.

Denn durch die einstückige Ausführung der Rotationstrommel ist es möglich die Rotationstrommel ohne Zwischenschritte oder zusätzlich notwendige Verbindungsteile direkt in der Lagergabel zu lagern. Hierdurch wird die Installation der Rotationstrommel erleichtert und zum Beispiel auch ermöglicht, die Rotationstrommel zum Testen der Rotation an einer externen Lagergabel zu installieren, ohne Einbindung in ein Endoskop. Da keine weiteren Bauteile neben der Rotationstrommel für die Installation nötig sind, wird entsprechend auch der notwendige Bauraum im distalen Ende des Endoskops minimiert, der für die Installation der Rotationstrommel nötig ist. Weiterhin wird durch die einstückige Ausführung ermöglicht, durch ein, bevorzugt mechanisches, Steuermittel ein Rotationsmoment direkt auf die zu rotierende Rotationstrommel zu übertragen, ohne dass die Kraft vorher über Verbindungselemente geleitet werden muss, wodurch ein Kraftverlust, bei gleichzeitig relativ simplem Aufbau, vermieden werden kann. Außerdem ermöglicht der Steuerfortsatz eine direkte oder unmittelbare Anbindung oder Verbindung mit dem Steuermittel, so dass auch die Fertigung im Zusammenhang mit dem Übergang zwischen Rotationstrommel und Steuermittel effektiv und mit wenig Fertigungsaufwand erfolgen kann.

Die Rotationstrommel besteht bevorzugt aus einem soliden und wenigstens teilweise elastisch verformbaren Material, welches für Fließpressverfahren geeignet ist, wie zum Beispiel nichtrostender Stahl oder Titan. Alternativ sind zum Beispiel auch Ausführungen aus Plastik oder Kunststoff denkbar. Hierdurch wird erreicht, dass der Steuerfortsatz die durch ein Steuermittel ausgeübte Kraft in ein Drehmoment umsetzen und sich dabei verformen oder verbiegen kann und gleichzeitig als federndes Rückstellmittel funktioniert, welche Rückkehr der Rotationstrommel in eine definierte Ausgangsposition bewirkt oder wenigstens unterstützt. Die Rotationstrommel kann als fest verbautes Bauteil ausgeführt sein, oder als auswechselbares Bauteil oder Rotationsmodul. Für die auswechselbare Variante kann optional eine zusätzliche Ausführung als Einwegbauteil vorgesehen sein.

Das Abbildungssystem besteht wenigstens aus einer Abbildungsoptik und einem Bildsensor. Die Abbildungsoptik kann zum Beispiel ein Objektiv sein, welches bevorzugt für den jeweiligen Anwendungszweck gewählt und ausgetauscht werden kann. Der Bildsensor kann zum Beispiel als "Charged-Coupled Device" (CCD) oder "Complementary Metal-Oxide-Semiconductor" (CMOS) ausgebildet sein. Besonders bevorzugt ist der Bildsensor als "Chip-on-the-Tip" ausgebildet. Andere Ausführungen der Abbildungsoptik und des Bildsensors sind möglich.

Die flexible Leiterplatte ist bevorzugt mit dem Bildsensor datenübertragend und/oder energieübertragend verbunden und verbindet bevorzugt den distalen Bereich mit dem proximalen Bereich des Endoskops. Die flexible Leiterplatte ist bevorzugt als "Printed Circuit Board" (PCB) ausgebildet und besteht aus flexiblem Material wie zum Beispiel Polyamid.

Die Steuermittel des Endoskops bestehen bevorzugt aus einem soliden Material, zum Beispiel Metall, und sind zum Beispiel als zylindrischer Draht ausgebildet. Diese Ausführung als Draht ist besonders platzsparend, weiterhin ist aber auch eine flächige Ausführung der Steuermittel denkbar, zum Beispiel als Steuerplatte. Bevorzugt umfasst das Endoskop wenigstens ein Steuermittel, es kann aber auch eine Vielzahl von Steuermitteln vorliegen, welche gleichartig, andersartig oder in einer Kombination aus gleich- und andersartigen Steuermitteln ausgebildet werden.

Gemäß einer ersten vorteilhaften Ausführungsform kann vorgesehen sein, dass das distale Ende des Endoskops zumindest eine Beleuchtungsvorrichtung umfasst, die derart angeordnet ist, dass wenigstens das durch die Rotationstrommel und deren Rotationswinkel eingestellte Sichtfeld beleuchtet ist, und/oder der gesamte durch Rotation mögliche Sichtbereich des Abbildungssystems beleuchtet ist.

Die Beleuchtungsvorrichtung kann bevorzugt mit der flexiblen Leiterplatte verbunden sein oder eine eigenständige Steuerung und/oder Energieversorgung aufweisen. Für die Beleuchtungsvorrichtung können zum Beispiel LEDs verwendet werden. Die Beleuchtungsvorrichtung kann am Schaftrohr des Endoskops ausgebildet sein oder an der Rotationstrommel. Weiterhin kann auch eine Vielzahl von Beleuchtungsvorrichtung vorgesehen sein, welche sowohl am Schaft als auch an der Rotationstrommel ausgebildet sind Die Anordnung der Beleuchtungsvorrichtung am Endoskop ist vorteilhaft da insbesondere bei minimal invasiven Eingriffen auf zusätzliche Lichtquellen verzichtet werden kann und die Orientierung der Beleuchtung auf die gewünschte Stelle einfach und intuitiv gewährleistet werden kann.

Gemäß einer weiteren vorteilhaften Ausführungsform kann vorgesehen sein, dass das Steuermittel, mit dem Steuerfortsatz kraftschlüssig oder stoffschlüssig verbunden ist, insbesondere verlötet oder verschweißt ist, wodurch lineare Bewegungen des Steuermittels, insbesondere Drücken und Ziehen des Steuermittels entlang des Schaftrohrs, insbesondere über eine elastische verformende Auslenkung des Steuerfortsatzes, in eine Rotationsbewegung der Rotationstrommel übersetzt werden.

Durch die kraftschlüssige Verbindung des Steuerfortsatz mit dem Steuermittel wird eine simple und stabile Konstruktion erreicht, um die Rotation der Rotationstrommel zu steuern. Weiterhin wird durch die direkte Kraftübertragung vom Steuermittel auf den Steuerfortsatz verhältnismäßig wenig Kraft benötigt um die Rotation zu erreichen, wodurch keine leistungsstarken Antriebsmittel für das Steuermittel nötig sind und zum Beispiel auch eine manuelle Steuerung möglich ist. Durch den monolithisch mit der Rotationstrommel verbundenen und wenigstens teilelastischen Steuerfortsatz wird eine einfache Konstruktion mit einer minimalen Anzahl an Bauteilen und beweglich aneinander oder zueinander gelagerten Teilen erreicht. Gemäß einer weiteren vorteilhaften Ausführungsform kann vorgesehen sein, dass die Rotationstrommel wenigstens einen, bevorzugt zwei, Achszapfen aufweist, welche bevorzugt monolithisch an der Rotationstrommel ausgebildet sind, über welche sie an der Lagergabel rotierbar, bevorzugt austauschbar, gelagert ist.

Die Achszapfen sind bevorzugt zylindrisch ausgebildet, weitere Ausführungsformen wie zum Beispiel kegelförmig sind allerdings ebenso möglich. Durch die bewegliche Lagerung an zwei Achszapfen kann besonders einfach eine gleichmäßige Rotation der Rotationstrommel erreicht werden. Die einstückige Ausführung gewährt dabei einen stabilen Halt der Achszapfen an der Lagerstelle, welcher auch nach einer Vielzahl von durchgeführten Rotationen erhalten bleibt. Weiterhin ermöglichen die Achszapfen ein einfaches Auslösen der Rotationstrommel aus der Lagergabel, wenn ein Austauschen der Rotationstrommel nötig ist.

Gemäß einer weiteren vorteilhaften Ausführungsform kann vorgesehen sein, dass der Steuerfortsatz derart an einem Körper der Rotationstrommel ausgebildet ist, dass ein axialer Mittelpunktswinkel von etwa 90° eingeschlossen wird, wodurch die Rotationstrommel um 0 bis 90° entlang der Rotationsachse rotierbar ist.

Dieser axiale Mittelpunktswinkel, welcher die maximale Rotation der Rotationstrommel festlegt, kann in der Ausführung um einige Grad variieren. Ein Winkel von etwa 90° hat sich als besonders vorteilhaft herausgestellt, um ein möglichst weites Sichtfeld bei geringer Materialabnutzung zu ermöglichen.

Gemäß einer weiteren vorteilhaften Ausführungsform kann vorgesehen sein, dass die flexible Leiterplatte gebogen, bevorzugt um 180°, am Bildsensor befestigt ist. Hierdurch wird vorteilhaft erreicht, dass die flexible Leiterplatte während der Rotation entrollt wird ohne auf Zug angespannt zu werden, wodurch die flexible Leiterplatte weniger Materialverschleiß erfährt. Ebenso gewährleistet diese Anordnung, dass die flexible Leiterplatte während der Rotation nicht mit anderen Bauteilen verhakt.

Gemäß einer weiteren vorteilhaften Ausführungsform kann vorgesehen sein, dass die flexible Leiterplatte auf einer vom Schaftrohr abgewandten Seite der Rotationstrommel mit dem Bildsensor verbunden ist.

Hierdurch kann ebenfalls vorteilhaft erreicht werden, dass die flexible Leiterplatte bei einer Rotationsbewegung entrollt wird, und weniger Materialverschleiß erfährt, als eine flexible Leiterplatte, welche auf Zug angespannt wird.

Gemäß einer weiteren vorteilhaften Ausführungsform kann vorgesehen sein, dass die Rotationstrommel einen Durchmesser von 2 bis 8 mm aufweist, bevorzugt 2 bis 3 mm.

Hierdurch wird erreicht, dass die Rotationstrommel bei gängigen Endoskop-Größen, zum Beispiel 4 mm oder 10 mm eingesetzt werden kann. Weiterhin hat sich diese Größendimension für die Bearbeitung und Herstellung der Rotationstrommel als besonders vorteilhaft herausgestellt.

Gemäß einer weiteren vorteilhaften Ausführungsform kann vorgesehen sein, dass der Steuerfortsatz eine Materialdicke von 30 bis 150 µm aufweist, bevorzugt 30 bis 60 µm und/oder eine Länge von 4 bis 15 mm aufweist, bevorzugt 4 bis 6 mm. Diese Materialdicke und Länge haben sich als besonders vorteilhaft herausgestellt, um die federartigen Eigenschaften des Steuerfortsatzes mit einer Stabilität, auch bei häufiger Rotation, zu vereinen.

Gemäß einer weiteren vorteilhaften Ausführungsform kann vorgesehen sein, dass das Steuermittel einen Durchmesser von 200 bis 1000 µm aufweist, bevorzugt 200 bis 400 µm.

In diesem Größenbereich kann das Steuermittel besonders stabil ausgebildet und mit dem Steuerfortsatz verbunden werden. Weiterhin ermöglicht diese Ausführung eine platzsparende Führung des Steuermittels entlang der Längsrichtung des Endoskops, wobei die Übertragung der am proximalen Ende des Endoskops ausgelösten Steuerung auf den Steuerfortsatz sicher gewährleistet bleibt.

Gemäß einer weiteren vorteilhaften Ausführungsform kann vorgesehen sein, dass der Steuerfortsatz in einem entspannten Zustand der Rotationstrommel, in welchem keine oder nur eine unwesentliche Rotation der Rotationstrommel vorliegt, wenigstens teilweise die Kreisbahn der Rotationstrommel weiterführt und/oder wenigstens teilweise senkrecht in Relation zur Drehachse des Rotationstrommelkörpers angeordnet ist.

Hierdurch wird der Steuerfortsatz besonders platzsparend an der Rotationstrommel ausgebildet und lässt sich gleichzeitig einfach mit Bauteilen, welche entlang der Längsrichtung des Endoskops angeordnet sind, wie zum Beispiel die Steuermittel, verbinden. Weiterhin wird durch die teilweise annähernd die Kreisbahn der Rotationstrommel weiterführende Anordnung des Steuerfortsatzes eine flüssige Rotationsbewegung begünstigt, wodurch der Materialverschleiß am Rotationslager gesenkt wird. Gemäß einer weiteren vorteilhaften Ausführungsform kann vorgesehen sein, dass die Rotationstrommel und der Steuerfortsatz im entspannten Zustand der Rotationstrommel im weitesten Sinne annähernd eine e-Form ausbilden.

Hierdurch wird die besonders platzsparende Ausführung der Rotationstrommel weiter begünstigt. Weiterhin hat sich diese Form als besonders robust für die Anordnung des Steuerfortsatzes herausgestellt.

Gemäß einer weiteren vorteilhaften Ausführungsform kann vorgesehen sein, dass der Steuerfortsatz wenigstens teilweise verformbar ausgebildet ist und im maximal rotierten Zustand der Rotationstrommel, vollständig oder annähernd senkrecht in Relation zur Drehachse des Rotationstrommelkörpers angeordnet ist.

Durch das "Entrollen" des Steuerfortsatzes, bis zu einer annähernd senkrecht in Relation zur Drehachse des Rotationstrommelkörpers angeordneten Position, wird auf besonders vorteilhafte Weise erreicht, dass die Rotationstrommel entlang ihrer Kreisbahn rotiert wird. Dabei wird durch die Verformbarkeit des Steuerfortsatzes gewährleistet, dass die Rotationsbewegung besonders flüssig, insbesondere ohne ruckartige Bewegungen stattfindet.

Gemäß einer weiteren vorteilhaften Ausführungsform kann vorgesehen sein, dass der Rotationstrommelkörper einen Durchmesser von 2 bis 8 mm aufweist, bevorzugt 2 bis 3 mm und bevorzugt halbkreisförmig ausgestaltet ist.

Diese Größe des Rotationstrommelkörpers hat sich als besonders vorteilhaft für die monolithische Umsetzung der Rotationstrommel für gängige Endoskop-Größen, zum Beispiel 4 mm und 10 mm herausgestellt. Die halbkreisförmige Ausführung des Rotationstrommelkörpers begünstigt hierbei besonders die Robustheit der Rotationstrommel und die Stabilität, insbesondere Flüssigkeit, der Rotationsbewegung.

Nachfolgend wird die vorliegende Erfindung anhand von lediglich schematischen, beispielhaften Ausführungsformen der Erfindung zeigenden Zeichnungen näher erläutert.
Fig. 1: eine schematische Querschnittsdarstellung einer Seitenansicht des distalen Bereichs eines Endoskops in einer ersten Ausführungsform
Fig. 2 eine schematische Querschnittsdarstellung einer Draufsicht eines Endoskops in einer ersten Ausführungsform
Fig. 3 eine perspektivische Seitenansicht des distalen Bereichs eines Endoskops in einer ersten Ausführungsform, ohne Gehäuse
Fig. 4 eine perspektivische Ansicht einer Rotationstrommel in einer ersten Ausführungsform

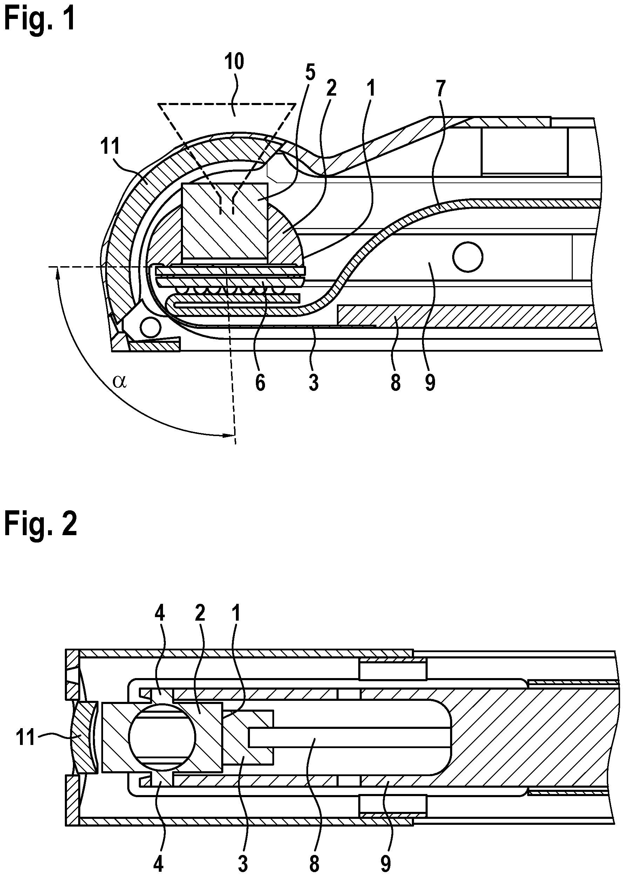

Die Figur 1 zeigte eine Querschnittsdarstellung einer Seitenansicht einer Ausführung des distalen Bereichs eines erfindungsgemäßen Endoskops. Bezüglich einer Darstellung und Beschreibung eines vollständigen gattungsgemäßen Endoskops kann zum Beispiel auf die DE 10 2020 132 778 A1 der Anmelderin und die dortige Figur 1a und 1b samt dazugehöriger Figurenbeschreibungen verwiesen werden.

Das Endoskop umfasst eine Lagergabel 9, welche zum Beispiel aus Metall oder Plastik/Kunststoff besteht. An der Lagergabel 9 ist eine Rotationstrommel 1 über bevorzugt zwei Achszapfen 4 beweglich gelagert. Die Achszapfen 4 sind monolithisch an der Rotationstrommel ausgebildet und sind bevorzugt zylindrisch ausgebildet, wobei andere Formen ebenfalls möglich sind. Die Rotationstrommel 1 umfasst einen Rotationstrommelkörper 2 mit einer Aussparung, in welche eine Abbildungsoptik 5, zum Beispiel ein Objektiv, aufgenommen werden kann. Weiterhin ist am Rotationstrommelkörper 2 ein Bildsensor 6 angeordnet, insbesondere ein "Charged-Coupled Device" (CCD) oder "Complementary Metal-Oxide-Semiconductor" (CMOS). Am Bildsensor 6 ist eine flexible Leiterplatte 7, insbesondere ein "Printed Circuit Board" (PCB), um 180° gebogen angeordnet. Bei einer Rotation der Rotationstrommel 1, kann sich die flexible Leiterplatte 7 entrollen und wird nicht auf Zug angespannt, wodurch der Materialverschleiß gesenkt wird.

Die Rotationstrommel 1 umfasst des Weiteren einen Steuerfortsatz 3, welcher monolithisch am Rotationstrommelkörper 2 ausgebildet ist. Der Steuerfortsatz 3 ist bevorzugt federnd ausgebildet und schließt einen axialen Mittelpunktswinkel α von 90° ein, um welchen die Rotationtrommel 1 rotiert werden kann. Ein Steuermittel 8 ist mit dem Steuerfortsatz 3 kraftschlüssig oder stoffschlüssig verbunden, insbesondere verlötet. Das Steuermittel 8 verläuft vom distalen zum proximalen Bereich des Endoskops und kann ein Signal zur Rotation der Rotationstrommel 1 vom proximalen Bereich auf den Steuerfortsatz 3 übertragen. Der Steuerfortsatz 3 ist bevorzugt wenigstens teilweise verformbar ausgebildet und ist bei maximaler Rotation der Rotationstrommel 1 annähernd senkrecht in Relation zur Rotationsachse angeordnet. Durch Rotation der Rotationstrommel 1 kann das Sichtfeld 10 um bis zu 90° rotiert werden.

Das Endoskop umfasst in seinem distalen Bereich des Weiteren ein Deckplättchen 11, welches bevorzugt aus Glas oder transparentem Plastik besteht und wenigstens den Sichtbereich abdeckt, bevorzugt einen Bereich, der wenigstens teilweise über das mögliche Sichtfeld hinausgeht, abdeckt. In der Figur 2 wird eine Querschnittsdarstellung einer Draufsicht einer Ausführung des distalen Bereichs eines erfindungsgemäßen Endoskops gezeigt. Das Endoskop umfasst auch hier eine Lagergabel 9, an welcher eine Rotationstrommel 1 über bevorzugt, 2 Achszapfen 4 beweglich gelagert ist. Die Achszapfen 4 sind monolithisch und bevorzugt zylindrisch an der Rotationstrommel 1 ausgebildet, wobei andere Formen ebenfalls möglich sind. Die Rotationstrommel 1 umfasst einen Rotationstrommelkörper 2 mit einer Aussparung. Ebenfalls monolithisch an der Rotationstrommel 1 ausgebildet ist der Steuerfortsatz 3, an welchem ein Steuermittel 8 kraftschlüssig angeordnet, insbesondere verlötet ist. Das Steuermittel 8 kann eine Ansteuerung der Rotationstrommel 1 vom proximalen zum distalen Bereich des Endoskops leiten um eine Rotation zu bewirken und steuern. Am distalen Ende des Endoskops ist weiterhin ein Deckplättchen 11 angeordnet, welches wenigstens den durch Rotation möglichen Sichtbereich umfasst.

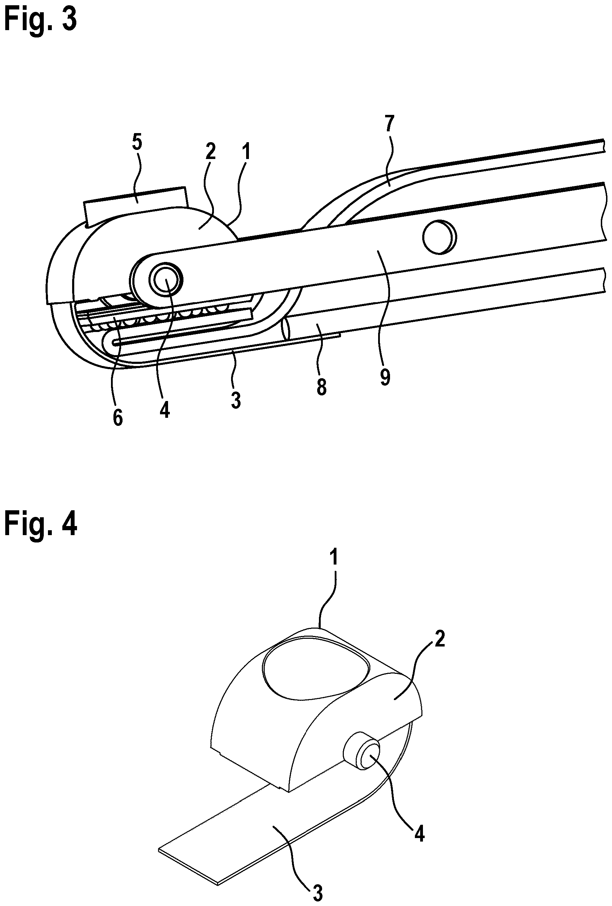

In der Figur 3 wird eine perspektivische Ansicht des distalen Bereichs eines erfindungsgemäßen Endoskops ohne ein umschließendes Gehäuse gezeigt. Das Endoskop umfasst eine Lagergabel 9, welche zum Beispiel aus Metall oder Plastik/Kunststoff besteht. An der Lagergabel 9 ist eine Rotationstrommel 1 über bevorzugt zwei Achszapfen 4 beweglich gelagert. Die Achszapfen 4 sind monolithisch an der Rotationstrommel ausgebildet und sind bevorzugt zylindrisch ausgebildet, wobei andere Formen ebenfalls möglich sind. Die Rotationstrommel 1 umfasst einen Rotationstrommelkörper 2 mit einer Aussparung, in welche eine Abbildungsoptik 5, zum Beispiel ein Objektiv, aufgenommen werden kann. Weiterhin ist am Rotationstrommelkörper 2 ein Bildsensor 6 angeordnet. Am Bildsensor 6 ist eine flexible Leiterplatte 7 um 180° gebogen angeordnet, welche den distalen Bereich des Endoskops mit dem proximalen Bereich, insbesondere datenübertragend, verbindet. Bei einer Rotation der Rotationstrommel 1, kann sich die flexible Leiterplatte 7 entrollen und wird nicht auf Zug angespannt, wodurch der Materialverschleiß gesenkt wird. Hierbei ist besonders gut zu sehen, dass bei einem Ziehen des Steuermittel 8, also einer Bewegung des Steuermittel 8 in die proximale Richtung, das Entrollen der flexiblen Leiterplatte 7 ohne eine Kollision mit dem Steuermittel 8 oder dem Steuerfortsatz 3 stattfindet.

Die Rotationstrommel 1 umfasst des Weiteren einen Steuerfortsatz 3, welcher monolithisch und bevorzugt federnd am Rotationstrommelkörper 2 ausgebildet ist. Ein Steuermittel 8 ist mit dem Steuerfortsatz 3 kraftschlüssig oder stoffschlüssig verbunden, insbesondere verlötet. Das Steuermittel 8 verläuft vom distalen zum proximalen Bereich des Endoskops und kann ein Signal zur Rotation der Rotationstrommel 1 vom proximalen Bereich auf den Steuerfortsatz 3 übertragen. Der Steuerfortsatz 3 ist bevorzugt wenigstens teilweise verformbar ausgebildet und ist bei maximaler Rotation der Rotationstrommel 1 annähernd senkrecht in Relation zur Rotationsachse angeordnet.

In der Figur 4 wird beispielhaft eine Rotationstrommel gezeigt, welche einen Rotationstrommelkörper umfasst, an welchem monolithisch ein Steuerfortsatz 3 ausgebildet ist, welcher bevorzugt federt. Ebenfalls monolithisch ausgebildet sind Achszapfen 4 die eine Lagerung in einer Lagergabel ermöglichen.

### Bezugszeichen

- 1: Rotationstrommel
- 2: Rotationstrommelkörper
- 3: Steuerfortsatz
- 4: Achszapfen
- 5: Abbildungsoptik
- 6: Bildsensor
- 7: flexible Leiterplatte
- 8: Steuermittel
- 9: Lagergabel
- 10: Sichtfeld
- 11: Deckplättchen

## Patentansprüche

1. Endoskop mit einem starren oder flexiblen Schaftrohr,
mit einer Rotationstrommel (1), welche an einem distalen Ende um wenigstens eine Rotationsachse rotierbar gelagert ist,
wobei die Rotationstrommel (1) eine Ausnehmung umfasst, in welcher ein zugeordnetes Abbildungssystem wenigstens teilweise aufgenommen wird, welches zumindest eine Abbildungsoptik (5) und einen Bildsensor (6) umfasst,
wobei das Abbildungssystem durch eine Leiterplatte (7) mit dem proximalen Bereich des Endoskops leitend verbunden ist,
und wobei das Endoskop zumindest ein Steuermittel (8) aufweist, um die Rotationstrommel (1) zu bewegen,
wobei
die Rotationstrommel (1) einstückig ausgeführt ist und wenigstens einen Steuerfortsatz (3) aufweist **dadurch gekennzeichnet,**
**dass** das Steuermittel (8), mit dem Steuerfortsatz (3) kraftschlüssig oder stoffschlüssig verbunden ist, wodurch lineare Bewegungen des Steuermittels (8), Drücken und Ziehen des Steuermittels (8) entlang des Schaftrohrs, über eine elastische verformende Auslenkung des Steuerfortsatzes (3) in eine Rotationsbewegung der Rotationstrommel (1) übersetzt werden.

2. Endoskop nach Anspruch 1,
**dadurch gekennzeichnet,**
**dass** das distale Ende des Endoskops zumindest eine Beleuchtungsvorrichtung umfasst, die derart angeordnet ist, dass wenigstens das durch den Rotationswinkel eingestellte Sichtfeld (10) beleuchtet ist, und/oder der gesamte durch Rotation mögliche Sichtbereich des Abbildungssystems beleuchtet ist.

3. Endoskop nach einem der vorhergehenden Ansprüche,
**dadurch gekennzeichnet,**
**dass** die Rotationstrommel (1) wenigstens einen Achszapfen (4) aufweist, welche an der Rotationstrommel (1) ausgebildet sind, über welche sie an der Lagergabel (9) rotierbar gelagert ist.

4. Endoskop nach einem der vorhergehenden Ansprüche,
**dadurch gekennzeichnet,**
**dass** die Leiterplatte (7) als flexible Leiterplatte (7) ausgebildet ist.

5. Endoskop nach Anspruch 4,
**dadurch gekennzeichnet,**
**dass** die Leiterplatte (7) gebogen am Bildsensor (6) befestigt ist.

6. Endoskop nach Anspruch 4 oder 5,
**dadurch gekennzeichnet,**
**dass** die Leiterplatte (7) auf einer vom Schaftrohr abgewandten Seite der Rotationstrommel (1) mit dem Bildsensor (6) verbunden ist.

7. Endoskop nach einem der vorhergehenden Ansprüche,
**dadurch gekennzeichnet,**
**dass** die Rotationstrommel (1) einen Durchmesser von 2 bis 8 mm aufweist.

8. Endoskop nach einem der vorhergehenden Ansprüche,
**dadurch gekennzeichnet,**
**dass** der Steuerfortsatz (3) eine Materialdicke von 30 bis 150 µm aufweist, und/oder eine Länge von 4 bis 15 mm aufweist.

9. Endoskop nach einem der vorhergehenden Ansprüche,
**dadurch gekennzeichnet,**
**dass** das Steuermittel (8) einen Durchmesser von 200 bis 1000 µm aufweist.

10. Endoskop nach einem der vorhergehenden Ansprüche,
**dadurch gekennzeichnet,**
**dass** der Steuerfortsatz (3) in einem entspannten Zustand der Rotationstrommel (1), in welchem keine oder nur eine unwesentliche Rotation der Rotationstrommel (1) vorliegt, wenigstens teilweise die Kreisbahn der Rotationstrommel (1) weiterführt und/oder wenigstens teilweise senkrecht in Relation zur Drehachse eines Rotationstrommelkörpers (2) der Rotationstrommel (1) angeordnet ist.

11. Endoskop nach einem der vorhergehenden Ansprüche,
**dadurch gekennzeichnet,**
**dass** die Rotationstrommel (1) und der Steuerfortsatz (3) im entspannten Zustand der Rotationstrommel (1) im weitesten Sinne annähernd eine e-Form ausbilden.

12. Endoskop nach einem der vorhergehenden Ansprüche,
**dadurch gekennzeichnet,**
**dass** ein Rotationstrommelkörper (2) der Rotationstrommel (1) einen Durchmesser von 2 bis 8 mm aufweist.
